# EUROPEAN PATENT APPLICATION

(11) **EP 3 944 782 A1**
(43) Date of publication of application: **02.02.2022**
(21) Application number: 20217131.0
(22) Date of filing: 23.12.2020
(51) Int. Cl.: A43B 1/00, A43B 17/00, A43B 13/38, A43B 7/00, A43B 7/14, A61H 39/00, A61H 39/04

(54) **MAGNETIC ACUPOINT MASSAGE INSOLE FOR CONDITIONING CHRONIC DISEASES**

(30) Priority: 28.07.2020 CN 202021522878 U
(71) Applicant: Guangdong Breath Walker Health Technology Co., Ltd., Dongguan City, Guangdong Province (CN)
(72) Inventor: ZHOU, Yuyuan, Dongguan City, Guangdong Province (CN); WU, Xingliang, Dongguan City, Guangdong Province (CN)
(74) Representative: Cabinet Chaillot

(57) **Abstract**

A magnetic acupoint massage insole for conditioning chronic diseases includes a left insole and a right insole. The left insole includes a heel (30), a middle part (20) and a head (10), and a magnet, a functional bump and an auxiliary bump are disposed on the left insole. The right insole comprises a heel (30a), a middle part (20a) and a head (10a), the right insole, and a magnet, a functional bump and an auxiliary bump are disposed on the right insole. The present invention has the effects of providing a better massage effect by the distribution of reasonably designed bump massage points together with the magnetic therapy effect provided by a magnet, stimulating the nerve endings of human body and organs, promoting user's blood circulation, and enhancing metabolism. In addition, the invention has a health care effect on a multiple of reflection zones, a high practicality, and a strong promotion effect.

## Description

### BACKGROUND OF THE INVENTION

### Technical Field

The present invention relates to wearing items, and more particularly to a magnetic acupoint massage insole for conditioning sub-health diseases and increasing immunity.

### Description of the Related Art

Human sole has more than 70 acupuncture points (abbreviated to "acupoints") and 6 meridians beginning and ending on a foot. Scientists believe that human sole has thousands of peripheral nerves closely connected with the brain and heart as well as different parts and organs of the human body, so that the feet are considered the "second heart" of human being, and a foot reflection zone refers to an area of a foot having a correspondence with a particular organ or part of a human body and capable of providing physiological and pathological information of the corresponding organ. By massaging and stimulating such reflection zone, the effect of adjusting the functions of various parts and organs of the human body, and conditioning sub-health diseases such as high blood pressure, diabetes, insomnia, constipation, varicose veins, etc. can be achieved.

In general, the structure of a conventional insole is simple and composed of a breathable surface and an elastic material layer attached to the bottom of the breathable surface. This kind of massage insoles can only serve as a simple foot rest, but cannot be used to massage the acupoints of a sole, and thus the conventional massage insoles do not have the magnetic therapy effect and cannot meet the needs of the users.

### SUMMARY OF THE INVENTION

Therefore, it is a primary objective of the present invention to provide an insole capable of conditioning sub-health diseases and increasing immunity.

To achieve the aforementioned and other objectives, the present invention discloses a magnetic acupoint massage insole for conditioning chronic diseases, and the magnetic acupoint massage insole comprises a left insole and a right insole, and the left insole comprises a heel, a middle part and a head, and the left insole further comprises a magnet, a functional bump and an auxiliary bump, a protrusion formed on the head of the left insole, and a first mounting hole formed at a central cross position of the protrusion and installed with the magnet, and a six bump disposed at a left position of the protrusion;
the head of the left insole comprises a plurality of first bumps disposed at both front side positions of the protrusion, and a second mounting hole formed on the head and at the right front position of the protrusion and installed with the magnet;
the middle part of the left insole has a heart-shaped convex block disposed at a central position of the middle part, and a third mounting hole formed on the convex block and installed with the magnet;
the middle part of the left insole comprises a fourth mounting hole disposed on the left side of the third mounting hole, a fifth mounting hole formed at a front position of the fourth mounting hole, and a sixth mounting hole and a seventh mounting hole formed at right positions of the middle part and arranged at the front and rear respectively, wherein the fourth mounting hole, the fifth mounting hole, the sixth mounting hole, and the seventh mounting hole are installed with the magnet respectively, and the left insole has a plurality of second bumps disposed around the periphery of the seventh mounting hole;
the heel of the left insole comprises an inner waist disposed at a position proximate to the front end of the heel, an eighth mounting hole formed on a recessed surface and at a position proximate to the middle of the rear of the heel and installed with the magnet, and a plurality of third bumps disposed around the periphery of the heel, a ninth mounting hole and a tenth mounting hole formed on left and right positions of the inner waist of the heel respectively, and the ninth mounting hole and the tenth mounting hole are installed with the magnet separately, a plurality of fourth bumps disposed around the periphery of the tenth mounting hole, and a plurality of fifth bumps disposed at the front position of the tenth mounting hole on the inner waist, wherein the bumps are functional bumps;
the right insole comprises a heel, a middle part and a head, and the right insole further comprises a magnet, a functional bump and an auxiliary bump, a protrusion formed on the head of the right insole, a first mounting hole formed at a central cross position of the protrusion and installed with the magnet, and a sixth bump disposed at a right position of the protrusion;
the head of the right insole comprises a plurality of first bumps disposed on the front positions of the protrusion, a second mounting hole formed at a front left position of the protrusion and installed with the magnet;
the middle part of the right insole comprises a heart-shaped convex block disposed at a central position of the middle part of the right sole, and a third mounting hole formed on the convex block and installed with the magnet;
the middle part of the right insole further comprises a sixth mounting hole and a seventh mounting hole formed at front and rear positions on the left side of the middle part respectively, and a fifth mounting hole formed at a right front position of the middle part of the right insole, wherein the fifth mounting hole, the sixth mounting hole, and the seventh mounting hole are installed with the magnet separately, and the right insole has a plurality of second bumps disposed around the periphery of the seventh mounting hole; and
the heel of the right insole comprises an eighth mounting hole formed on a recessed surface and at a position proximate to the middle of a rear position of the heel of the right insole and installed with the magnet, a plurality of third bumps disposed around the periphery of the eighth mounting hole of the heel of the right insole, a ninth mounting hole and a tenth mounting hole formed at both right and left positions of the inner waist of the heel of the right insole respectively, wherein the ninth mounting hole and the tenth mounting hole are installed with a magnet separately, and the heel has a plurality of fourth bumps disposed around the periphery of the tenth mounting hole, and the inner waist has a plurality of fifth bumps disposed at a front position of the tenth mounting hole.

Further, the protrusion of the left insole is substantially in a herringbone shape, and the thickness of the protrusion is tapered from the central cross position towards both ends.

Further, the middle of the heel of the left insole is recessed, and the edge portion of the heel is upwardly warped to form a foot cup design.

Further, the protrusion of the right insole is substantially herringbone shaped, and the thickness of the protrusion is tapered from the central cross position towards both ends.

Further, the middle of the heel of the right insole is recessed, and the edge portion of the heel is upwardly warped to form a foot cup design.

Further, the left insole and right insole massage bumps are in an elongated shape and extend in left and right directions respectively.

The present invention has the advantageous effects of providing a better massage effect by the distribution of reasonably designed bump massage points together with the magnetic therapy effect provided by a magnet, stimulating the nerve endings of human body and organs, promoting user's blood circulation, and enhancing metabolism. In addition, the invention has a health care effect on a multiple of reflection zones, a high practicality, and a strong promotion effect.

### BRIEF DESCRIPTION OF THE DRAWINGS

FIG. 1 is a top view of a left insole of a magnetic acupoint massage insole for conditioning chronic diseases in accordance with the present invention; and
FIG. 2 is a top view of a right insole of a magnetic acupoint massage insole for conditioning chronic diseases in accordance with the present invention.

### DESCRIPTION OF THE PREFERRED EMBODIMENTS

The technical solution of the present invention will become apparent from the following detailed description taken with the accompanying drawings.

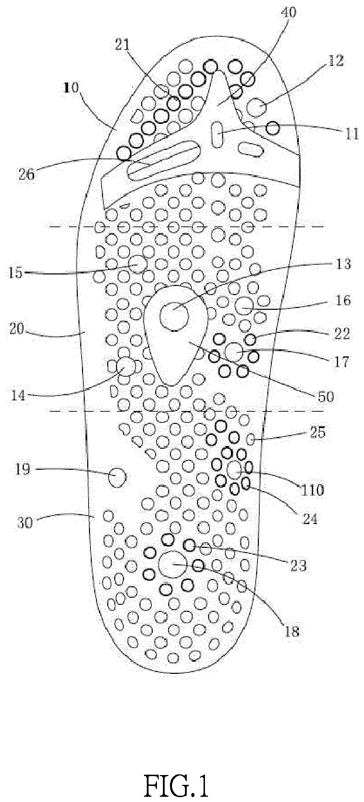

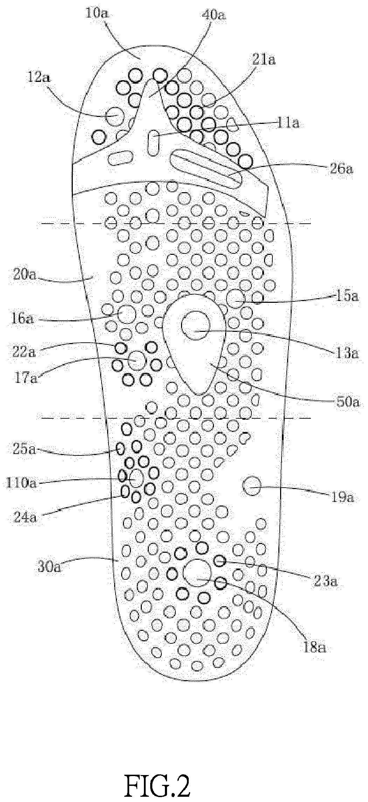

With reference to FIGS. 1 and 2 for a magnetic acupoint massage insole for conditioning chronic diseases in accordance with a preferred embodiment of the present invention, the magnetic acupoint massage insole comprises a left insole and a right insole, and a layer of contact surface covered onto upper surfaces (not shown in the figure) of the left insole and the right insole to protect a user's sole, and the structure of the left insole will be described first.

The left insole comprises a heel 30, a middle part 20 and a head 10 (as shown by three areas divided by a horizontal dotted line in FIG. 1 respectively). The left insole has a magnet, a functional bump and an auxiliary bump disposed on the left insole, and a mountain-shaped protrusion 40 is formed on the head 10 of the left insole. Specifically, the protrusion 40 is substantially herringbone shaped, and the thickness of the protrusion 40 is tapered from a central cross position towards both ends. The central cross position the protrusion 40 has a first mounting hole 11 for installing the magnet, and the magnet installed in the first mounting hole 11 acts on a trigeminal nerve reflection zone. The massage bumps are in an elongated shape and extend in left and right directions. The left position of the protrusion 40 has a sixth bump 26 which is a functional bump aligned with a human eye reflection zone.

The head 10 has a plurality of first bumps 21 disposed at both front side positions of the protrusion 40. Compared with a conventional insole, the front side positions of the head 10 are thickened by 2.0 mm to improve the massage of the user's frontal sinus by the first bumps 21 of the head 10 to increase human immunity. The head 10 has a second mounting hole 12 formed at the right front of the protrusion 40, and a magnet is installed to the second mounting hole 12 and aligned with a human pituitary gland reflection zone for conditioning human endocrine.

The central position of the middle part 20 of the left insole has a heart-shaped convex block 50, and a third mounting hole 13 is formed on the convex block 50 for installing the magnet to produce a magnetic induction to the Yong Quan Point of the user's sole.

The middle part 20 of the left insole has a fourth mounting hole 14 disposed on the left side of the third mounting hole 13, and the middle part 20 has a fifth mounting hole 15 formed at the front of the fourth mounting hole 14, and the right position of the middle part 20 has a sixth mounting hole 16 and seventh mounting hole 17 arranged at the front and rear respectively, and the fourth mounting hole 14, fifth mounting hole 15, sixth mounting hole 16, and seventh mounting hole 17 have a magnet installed thereto and aligned with a human spleen reflection zone, heart reflection zone, stomach reflection zone, and pancreas reflection zone respectively, and the left insole has a plurality of second bumps 22 disposed at the periphery of the seventh mounting hole 17 for strengthening the massage of the pancreatic gland and duodenum to promote digestion and regulate diabetes.

The middle of the heel 30 of the left insole is recessed, and the edge portion of the heel 30 is upwardly warped to form a foot cup design, so as to achieve a more stable arch support and prevent cricket, and an inner waist is formed at the heel 30 and disposed proximate to a front position of the heel 30; the heel 30 has an eighth mounting hole 18 formed on a recessed surface and at a positon proximate to the middle of the rear position of the heel 30 and installed with a magnet, and aligned with a human gonad reflection zone, and the heel 30 further comprises a plurality of third bumps 23 disposed around the periphery of the eighth mounting hole 18, wherein the bumps are functional bumps for increasing the massage strength for the gonad reflection zone. The heel 30 of the left insole further comprises a ninth mounting hole 19 and a tenth mounting hole 110 formed on left and right positions of the inner waist respectively and installed with a magnet separately, and aligned with a human colon reflection zone and a kidney reflection zone respectively, and a plurality of fourth bumps 24 disposed around the periphery of the tenth mounting hole 110, wherein the bumps are functional bumps for increasing the massage area of the kidney and adjusting urinary frequency and urgency, and the inner waist comprises has a plurality of fifth bumps 25 formed at a front position of the tenth mounting hole 110, wherein the bumps are functional bumps for increasing the massage points of an anus and conditioning hemorrhoid.

The right insole comprises a heel 30a, a middle part 20a and a head 10a (as shown by the three areas divided by the horizontal dotted line of FIG. 2 respectively). The right insole further comprises a magnet, and a functional bump and an auxiliary bump disposed thereon, and a mountain-shaped protrusion 40a is formed on the head 10a of the right insole. Specifically, the protrusion 40a is substantially in a herringbone shape, and the thickness of the protrusion 40a is tapered from a central cross position towards both ends. The protrusion 40a further comprises a first mounting hole 11a formed at the central cross position of the protrusion 40a and installed with a magnet, and the magnet of the first mounting hole 11a acts on a trigeminal nerve reflection zone; the massage bumps are substantially in an elongated shape and extend in left and right directions respectively. The protrusion 40a has a sixth bump 26a disposed at a right position of the protrusion 40a, wherein the bumps are functional bumps aligned with a human eye reflection zone.

The head 10a comprises a plurality of first bumps 21a disposed at both front side positions of the protrusion 40a. Compared with a conventional insole, the front side position of the head 10a is thickened by 2.0 mm to increase the massage of the frontal sinus at the position of the head 10a and increase human immunity. The head 10a has a second mounting hole 12a formed on the left front of the protrusion 40a and installed with a magnet, and aligned with a human pituitary gland reflection zone for adjusting human endocrine.

The middle part 20a of the right insole comprises a heart-shaped convex block 50a disposed at a central position thereof, a third mounting hole 13a formed on the convex block 50a and installed with a magnet to produce a magnetic induction to the Yong Quan Point of a user's sole.

The middle part 20a of the right insole comprises a sixth mounting hole 16a and a seventh mounting hole 17a formed at front and rear positions of the left side thereof, a fifth mounting hole 15a formed at a right front position of the middle part 20a of the right insole, wherein the fifth mounting hole 15a, the sixth mounting hole 16a, and the seventh mounting hole 17a are installed with a magnet separately and aligned with a human liver reflection zone, a stomach reflection zone, and a spleen reflection zone respectively, and the right insole further comprises a plurality of second bumps 22a disposed at the periphery of the seventh mounting hole 17a for strengthening the massage of the pancreatic gland and duodenum, promoting digestion, and regulating diabetes.

The middle of the heel 30a of the right insole is recessed, and the edge portion of the heel 30a is upwardly warped to form a foot cup design, so as to achieve a more stable arch support and prevent cricket, and an inner waist is formed at the heel 30 and disposed proximate to a front position of the heel 30; the heel 30a comprises an eighth mounting hole 18a formed on a recessed surface and at a position proximate to the rear side of the heel 30, and the eighth mounting hole 18a has a magnet installed therein and aligned with a human gonad reflection zone, and the heel 30a further has a plurality of third bumps 23a disposed around the periphery of the eighth mounting hole 18a, wherein the bumps are functional bumps for increasing the massage strength of the gonad. The heel 30a further comprises a ninth mounting hole 19a and a tenth mounting hole 110a formed at right and left positions of the inner waist respectively and installed with a magnet separately, and aligned with a human appendix reflection zone and a kidney reflection zone respectively, and the heel 30a further comprises a plurality of fourth bumps 24a disposed around the periphery of the tenth mounting hole 110a, wherein the bumps are functional bumps for increasing the massage area of the kidney, adjusting the urinary frequency and urgency, and the inner waist has a plurality of fifth bumps 25a disposed at a front position of the tenth mounting hole 110a, wherein the bumps are functional bumps for increasing the massage points of an anus and conditioning hemorrhoid.

The present invention has the advantageous effects of providing a better massage effect by the distribution of reasonably designed bump massage points together with the magnetic therapy effect provided by a magnet, stimulating the nerve endings of human body and organs, promoting user's blood circulation, and enhancing metabolism. In addition, the invention has a health care effect on a multiple of reflection zones, a high practicality, and a strong promotion effect.

While the invention is described in some detail hereinbelow with reference to certain illustrated embodiments, it is to be understood that there is no intent to limit the invention to those embodiments. On the contrary, the aim is to cover all modifications, alternatives and equivalents falling within the spirit and scope of the invention as defined by the appended claims.

## Claims

1. A magnetic acupoint massage insole for conditioning chronic diseases, comprising a left insole and a right insole, and the left insole comprising a heel (30), a middle part (20) and a head (10), and the left insole further comprising a magnet, a functional bump and an auxiliary bump disposed thereon, a protrusion (40) formed on the head (10) of the left insole, and a first mounting hole (11) formed at a central cross position of the protrusion (40) and provided for mounting the magnet, and a sixth bump (26) disposed at a left position of the protrusion (40); the head (10) comprising: a plurality of first bumps (21) disposed at both front side positions of the protrusion (40), a second mounting hole (12) formed at a right position of the protrusion (40) and having a magnet installed thereto; the middle part (20) comprising: a heart-shaped convex block (50) formed at the central position of the middle part (20) of the left insole, and the convex block (50) having a third mounting hole (13) formed thereon, and the magnet being installed to the third mounting hole (13); a fourth mounting hole (14) formed on the middle part (20) of the left insole and disposed on a left side of the third mounting hole (13); a fifth mounting hole (15) formed at the front of the fourth mounting hole (14), and a sixth mounting hole (16) and a seventh mounting hole (17) formed at the right position of the middle part (20) and arranged at the front and rear respectively, and the fourth mounting hole (14), the fifth mounting hole (15), the sixth mounting hole (16), and the seventh mounting hole (17) having a magnet separately, and the left insole having a plurality of second bumps (22) disposed at the periphery of the seventh mounting hole;
an inner waist formed at a position proximate to the front end of the heel (30) of the left insole; an eighth mounting hole (18) formed at a position proximate to the rear of the concave surface of the heel (30), and having a magnet, the heel (30) installed thereto, and a plurality of third bumps (23) disposed around the periphery of the eighth mounting hole (18); the heel (30) comprising a ninth mounting hole (19) and a tenth mounting hole (110) formed on both left and right sides of the inner waist respectively and the ninth mounting hole (19) and the tenth mounting hole (110) have a magnet installed thereto respectively, and the heel (30) further comprising a plurality of fourth bumps (24) disposed around the periphery of the tenth mounting hole (110), and the inner waist comprising a plurality of fifth bumps (25) disposed at a front position of the tenth mounting hole (110);
the right insole comprising a heel (30a), a middle part (20a) and a head (10a), and the right insole comprising a magnet, a functional bump and an auxiliary bump, and a protrusion (40a) formed on the head (10a) of the right insole, and the protrusion (40a) having a first mounting hole (11a) formed at a central cross position thereof, and the first mounting hole (11a) of the right insole being installed with the magnet, and the protrusion (40a) having a sixth bump (26a) disposed at a right position thereof;
the head (10a) of the right insole further comprising a plurality of first bumps (21a) formed at both front side positions of the protrusion (40a), and a second mounting hole (12a) formed on the front left of the protrusion (40a) and the second mounting hole (12a) having a magnet installed thereto;
the middle part (20a) of the right insole comprising a heart-shaped convex block (50a) disposed at the central position thereof, and the convex block (50a) comprising a third mounting hole (13a) installed with the magnet; and the middle part (20a) of the right insole further comprising a sixth mounting hole (16a) and a seventh mounting hole (17a)formed at the left front and left rear positions of the middle part (20a) of the right insole respectively, a fifth mounting hole (15a) formed at the right front position of the middle part (20a) of the right insole, and the fifth mounting hole (15a), the sixth mounting hole (16a), and the seventh mounting (17a) hole having a magnet installed thereto separately, and the right insole having a plurality of second bumps (22a) formed at the periphery of the seventh mounting hole;
the heel (30a) of the right insole comprising an eighth mounting hole (18a) formed on a recessed surface and proximate to the middle of a rear position thereof, and the eighth mounting hole (18a) having a magnet, a plurality of third bumps (23a) disposed around the periphery of the eighth mounting hole (18a), a ninth mounting hole (19a) and a tenth mounting hole (110a) formed at the right and left positions of the inner waist of the heel (30a) respectively, and the ninth mounting hole (19a) and the tenth mounting hole (110a) having a magnet installed thereto separately, a plurality of fourth bumps (24a) disposed around the periphery of the tenth mounting hole (110a) of the heel (30a), and a plurality of fifth bumps (25a) disposed at a front position of the tenth mounting hole (110a) of the inner waist.

2. The magnetic acupoint massage insole for conditioning chronic diseases as claimed in claim 1, wherein the protrusion (40) of the left insole is substantially in a herringbone shape, and the thickness of the protrusion (40) is tapered from the central cross position towards both ends.

3. The magnetic acupoint massage insole for conditioning chronic diseases as claimed in claim 1, wherein the middle of the heel (30) of the left insole is recessed, and the edge portion of the heel (30) is upwardly warped to form a foot cup design.

4. The magnetic acupoint massage insole for conditioning chronic diseases as claimed in claim 1, wherein the protrusion (40a) of the right insole is substantially herringbone shaped, and the thickness of the protrusion (40a) is tapered from the central cross position towards both ends.

5. The magnetic acupoint massage insole for conditioning chronic diseases as claimed in claim 1, wherein the middle of the heel (30a) of the right insole is recessed, and the edge portion of the heel (30a) is upwardly warped to form a foot cup design.

6. The magnetic acupoint massage insole for conditioning chronic diseases as claimed in claim 1, wherein the left insole and right insole massage bumps are in an elongated shape and extend in left and right directions respectively.
